# EUROPÄISCHE PATENTSCHRIFT

(11) **EP 0 562 343 B1**
(45) Veröffentlichungstag und Bekanntmachung des Hinweises auf die Patenterteilung: **15.11.1995**
(21) Anmeldenummer: 93103715.4
(22) Anmeldetag: 09.03.1993
(51) Int. Cl.: C07C 67/343, C07C 69/675

(54) **Verfahren zur Herstellung von beta-Hydroxycarbonsäureestern**
Process for the preparation of esters of beta-hydroxycarboxylic acids
Procédé de préparation d'esters d'acides bêta-hydroxycarboxyliques

(30) Priorität: 25.03.1992 DE 4209616
(43) Veröffentlichungstag der Anmeldung: 29.09.1993
(73) Patentinhaber: BASF Aktiengesellschaft, 67063 Ludwigshafen (DE)
(72) Erfinder: Paust, Joachim, Dr., W-6708 Neuhofen (DE); Siegel, Wolfgang, Dr., W-6800 Mannheim 1 (DE)

(56) Entgegenhaltungen:
- SYNTHESIS, Nr. 7, Juli 1975, Stuttgart, DE, Seiten 452 - 453; R.D.RIEKE: "Activated metals. XI. An improved procedure for the preparation of beta-hydroxy esters using activated zinc"

## Beschreibung

Die Erfindung betrifft ein Verfahren zur Herstellung von β-Hydroxycarbonsäureestern durch Umsetzung von Carbonylverbindungen mit α-Bromcarbonsäureestern und Zink in Methylenchlorid bei Temperaturen von 0 bis 50°C und die Verwendung von Methylenchlorid zur stabilen Lagerung von α-Bromozinkcarbonsäureestern.

α-Bromozinkcarbonsäureester (Reformatsky-Verbindungen) lassen sich mit einer Vielzahl von Elektrophilen z.B. Aldehyden, Ketonen, Azomethinen, Nitrilen, Carbonsäurechloriden, -anhydriden und -estern, Lactonen und Epoxiden zur Reaktion bringen und sind daher für die Herstellung von wichtigen Bausteinen für die organische Synthese und den Aufbau von Naturstoffen von Bedeutung.

Zur Erzielung guter Ausbeuten bei der Synthese von β-Hydroxycarbonsäureestern durch Umsetzung von Carbonylverbindungen mit α-Bromcarbonsäureestern und Zink (Reformatsky-Reaktion) ist es notwendig, das verwendete Zink oder die gesamte Reaktionsmischung mittels aufwendiger Verfahren zu aktivieren.

Als Lösungsmittel finden vorallem Ether wie Diethylether, Tetrahydrofuran, Dioxan, Ethylenglykoldimethylether, Dimethoxymethan, sowie Dimethylformamid, Dimethylsulfoxid, Acetonitril, Hexamethylphosphorsäuretriamid, Borsäuretrimethylester, aromatische Kohlenwasserstoffe wie Benzol und deren Mischungen Verwendung.

Die Wahl des Lösungsmittel ist für die sichere Durchführung der Reaktion von entscheidender Bedeutung, da die Bildung der α-Bromozinkcarbonsäureester (Reformatsky-Verbindung) sehr exotherm verläuft und bei Verwendung von zu hochsiedenden Lösungsmitteln eine kontrollierbare Reaktionsführung bei Reaktionsansätzen im technischen Maßstab nicht zuverlässig gewährleistet ist.

Aus Bull. Chem. Soc. 53, 3301 (1980), Tetrahedron Lett. 88, 6481 (1987), Synth. Comm. 17, 1 (1987) ist die Verwendung von Legierungen aus Zink/Silber zur Herstellung der α-Bromozinkcarbonsäureester bzw. bei der Reformatsky-Reaktion bekannt. Die Verwendung von in situ hergestelltem Zink aus der Reduktion von Zinkhalogeniden mit Alkalimetallen wird in Synthesis 452 (1975), Organometallics 5, 1257 (1986) und Ultraschall zur Aktivierung in J. Org. Chem. 47, 5030 (1982) empfohlen.

Diese Methoden zur Verbesserung der Ausbeute von Reformatsky-Reaktionen sind teuer und wegen des hohen Aufwands im technischen Maßstab nicht anwendbar.

Der vorliegenden Erfindung lag daher die Aufgabe zugrunde, den zuvor genannten Nachteilen abzuhelfen.

Demgemäß wurde ein neues und verbessertes Verfahren zur Herstellung von β-Hydroxycarbonsäureestern der allgemeinen Formel I 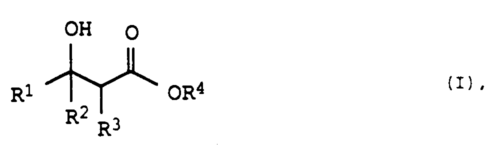
in der
- R¹, R²: gegebenenfalls durch C₁- bis C₈-Alkoxy und/oder Halogen substituiertes C₁- bis C₂₀-Alkyl, C₃- bis C₂₀-Alkoxycarbonyl-alkyl, C₂- bis C₂₀-Alkenyl, C₅- bis C₃₀-Acetalalkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₇- bis C₂₀-Aralkenyl, Hetaryl, C₃- bis C₂₀-Cycloalkyl, C₄- bis C₂₀-Cycloalkyl-alkyl oder gemeinsam eine C₂- bis C₈-Alkylenkette und R¹ oder R² Wasserstoff,
- R³: Wasserstoff, C₁- bis C₂₀-Alkyl, C₂- bis C₂₀-Alkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₃- bis C₂₀-Cycloalkyl oder C₄- bis C₂₀-Cycloalkyl-alkyl,
- R⁴: C₁- bis C₂₀-Alkyl, C₄- bis C₂₀-Alkenyl, C₇- bis C₂₀-Aralkyl, C₃- bis C₂₀-Cycloalkyl oder C₄- bis C₂₀-Cycloalkyl-alkyl,
bedeuten, durch Umsetzung von Carbonylverbindungen der allgemeinen Formel II 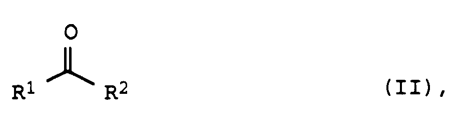
in der R¹ und R² die oben genannten Bedeutungen hat, mit α-Brom-carbonsäureestern der allgemeinen Formel III 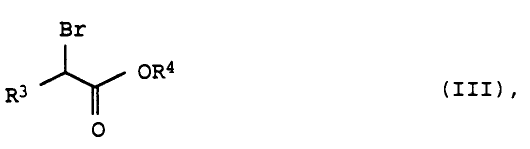
in der R³ und R⁴ die oben genannten Bedeutungen hat, gefunden, welches dadurch gekennzeichnet ist, daß man die Umsetzung mit Zink in Methylenchlorid bei Temperaturen von 0 bis 50°C durchführt sowie ein Verfahren zur Herstellung von α-Bromozinkcarbonsäureestern der allgemeinen Formel IV 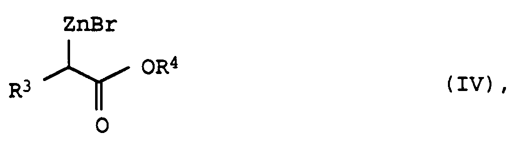
in der
- R³: Wasserstoff, C₁- bis C₂₀-Alkyl, C₂- bis C₂₀-Alkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₃- bis C₂₀-Cycloalkyl oder C₄- bis C₂₀-Cycloalkyl-alkyl,
- R⁴: C₁- bis C₂₀-Alkyl, C₄- bis C₂₀-Alkenyl, C₇- bis C₂₀-Aralkyl, C₃- bis C₂₀-Cycloalkyl oder C₄- bis C₂₀-Cycloalkyl-alkyl,
bedeuten, aus α-Bromcarbonsäureestern der allgemeinen Formel III, welches dadurch gekennzeichnet ist, daß man die Umsetzung mit Zink in Methylenchlorid bei Temperaturen von 0 bis 50°C durchführt und die Verwendung von Methylenchlorid zur stabilen Lagerung der α-Bromozinkcarbonsäureester IV.

Das erfindungsgemäße Verfahren kann wie folgt durchgeführt werden:
Man kann das Zink in Methylenchlorid vorlegen und den α-Bromcarbonsäureester unter Temperaturkontrolle zugeben, wobei sich lagerstabile Lösungen von α-Bromozinkcarbonsäureester IV bilden. Diese Lösungen können in situ mit einer Carbonylverbindung II durch dessen Zugabe ebenfalls unter Temperaturkontrolle zu den β-Hydroxycarbonsäureestern I umgesetzt werden.

Es ist auch möglich, das Zink in Methylenchlorid vorzulegen und eine Mischung aus α-Bromocarbonsäureester IV und einer Carbonylverbindung II unter Temperaturkontrolle zuzugeben.

Ferner ist es auch möglich, das Zink und die Carbonylverbindung vorzulegen und den α-Bromocarbonsäureester IV unter Temperaturkontrolle zuzugeben.

Die Temperaturkontrolle kann durch Kühlen der Reaktionsmischung erfolgen. Die obere Temperaturgrenze wird u.a. vom Lösungsmittel Methylenchlorid (Sdp. 42°C) bestimmt und beträgt ca. 40 bis 45°C. Die Reaktion wird in aller Regel bei Temperaturen von 0 bis 50°C durchgeführt, bevorzugt bei 20 bis 50°C, besonders bevorzugt bei 35 bis 45°C.

Der Druck bei der Reaktion ist unkritisch und kann in weiten Grenzen gewählt werden. In aller Regel arbeitet man bei 0,01 bis 50 bar bevorzugt bei 0,5 bis 5 bar, besonders bevorzugt bei Normaldruck (Atmosphärendruck).

Als Lösungsmittel findet Methylenchlorid Verwendung, das bevorzugt im wesentlichen in Abwesenheit von zusätzlichen Lösungsmitteln eingesetzt werden sollte.

Das Zink kann in Band-, Kugel-, Span- und Pulverform eingesetzt werden, bevorzugt verwendet man die Pulverform. Zink kann bevorzugt im wesentlichen in Abwesenheit anderer Metalle, wie edlere Metalle z.B. Kupfer und Silber eingesetzt werden, d.h. man setzt bevorzugt handelsübliches Zink ein.

Die β-Hydroxycarbonsäureester I lassen sich nach üblichen Methoden wie Extraktion, Destillation, Chromatographie und Kristallisation erhalten.

Die vorliegende Erfindung stellt ein allgemein anwendbares Verfahren zur Herstellung von Reformatsky-Verbindungen aus handelsüblichem Zink und α-Brom-Carbonylverbindungen und deren Umsetzung mit Elektrophilen in Methylenchlorid als Lösungsmittel dar.

Besonders vorteilhaft im Hinblick auf eine technische Anwendung ist die einfache Durchführbarkeit der Reformatsky-Synthese in Methylenchlorid, da weder die Reaktanten noch die Lösungsmittel speziell getrocknet werden müssen.

Die im allgemeinen als Einzelmaßnahme nicht allein ausreichende Aktivierung des Zinks z.B. mit einer katalytischen Menge Iod reicht in diesem Falle aus, um ausgezeichnete Ausbeuten und hohe Produktreinheit bei der Umsetzung mit Elektrophilen bei gleichzeitig sehr guten Raum-Zeit-Ausbeuten zu erreichen. Die bei der Bildung der Reformatsky-Verbindung freiwerdende Reaktionswärme kann z.B. mittels Siedekühlung durch das Methylenchlorid zuverlässig abgeführt und gewährleistet die sichere Reaktionsführung ohne hohen mess- und regeltechnischen Aufwand.

Die Substituenten R¹, R², R³ und R⁴ in den Formeln I, II, III und IV haben die folgenden Bedeutungen:
- R¹, R², R³, R⁴: - C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl,iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₂- bis C₂₀-Alkenyl, bevorzugt C₂- bis C₈-Alkenyl wie Vinyl, Allyl, But-2-en-1-yl, But-4-en-1-yl, But-4-en-2-yl, Pent-2-en-1-yl und 2,2-Dimethylpent-1-en-1-yl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₃- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Cycloalkyl-alkyl, bevorzugt C₄- bis C₁₂-Cycloalkyl-alkyl wie Cyclopentyl-methyl und Cyclohexyl-methyl,
- durch C₁- bis C₈-Alkoxy und/oder Halogen substituiertes C₁- bis C₂₀-Alkyl wie 2-Methoxy-ethyl, 3-Methoxy-propyl, 3-Methoxybutyl, 4-Methoxybutyl, Chlormethyl, 2-Chlorethyl und 3-Chlor-propyl,
- R¹, R², R³: - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- durch C₁- bis C₈-Alkyl, C₁- bis C₈-Alkoxy und/oder Halogen ein bis fünffach substituiertes Aryl, bevorzugt durch C₁- bis C₄-Alkyl, C₁- bis C₄-Alkoxy und/oder Halogen ein- bis dreifach substituiertes Aryl, wie 2-Alkylphenyl, 3-Alkylphenyl, 4-Alkylphenyl, 2,3-Dialkylphenyl, 2,4-Dialkylphenyl, 2,5-Dialkylphenyl, 2,4,6-Trialkylphenyl, 2-Alkoxyphenyl, 3-Alkoxyphenyl, 4-Alkoxyphenyl, 2,4-Dialkoxyphenyl und 4-Chlorphenyl, besonders bevorzugt Methyl, Ethyl, Methoxy, Ethoxy, Fluor und/oder Chlor ein- bis dreifach substituiertes Phenyl, wie 2-Methylphenyl, 3-Methylphenyl, 4-Methylphenyl, 4-Methoxyphenyl und 4-Chlorphenyl
- durch C₁- bis C₈-Alkyl, C₁- bis C₈-Alkoxy und/oder Halogen ein bis fünffach substituiertes C₇- bis C₂₀-Aralkyl, bevorzugt durch C₁- bis C₄-Alkyl, C₁- bis C₄-Alkoxy und/oder Halogen ein- bis dreifach substituiertes Aralkyl, wie 4-Alkylphenyl-alkyl, 4-Alkoxyphenyl-alkyl, 4-Chlorphenyl-alkyl und 3,5-Dialkylphenyl-alkyl, besonders bevorzugt Methyl, Ethyl, Methoxy, Ethoxy, Fluor und/oder Chlor ein- bis dreifach substituiertes Phenyl-alkyl, wie 4-Methylphenyl-methyl, 4-Methoxyphenyl-methyl und 4-Chlorphenyl-methyl,
- durch C₁- bis C₈-Alkyl, C₁- bis C₈-Alkoxy und/oder Halogen ein bis fünffach substituiertes C₃- bis C₂₀-Cycloalkyl, bevorzugt durch C₁- bis C₄-Alkyl, C₁- bis C₄-Alkoxy und/oder Halogen ein- bis dreifach substituiertes C₅- bis C₈-Cycloalkyl, wie 2-Alkyl-cyclohexyl, 4-Alkyl-cyclohexyl, 2-Alkoxy-cyclohexyl und 4-Alkoxy-cyclohexyl, besonders bevorzugt Methyl, Ethyl, Methoxy, Ethoxy, Fluor und/oder Chlor ein- bis dreifach substituiertes C₅- bis C₈-Cycloalkyl, wie 2-Methyl-cyclohexyl, 4-Methyl-cyclohexyl und 2-Methoxy-cyclohexyl,
- durch C₁- bis C₈-Alkyl, C₁- bis C₈-Alkoxy und/oder Halogen ein bis fünffach substituiertes C₄- bis C₂₀-Cycloalkyl-alkyl, bevorzugt durch C₁- bis C₄-Alkyl, C₁- bis C₄-Alkoxy und/oder Halogen ein- bis dreifach substituiertes C₆- bis C₁₂-Cycloalkyl-alkyl, wie 2-Alkylcyclohexylmethyl, 4-Alkylcyclohexylmethyl, 2-Alkoxy-cyclohexylmethyl, 4-Alkoxy-cyclohexylmethyl, 2-Halogen-cyclohexylmethyl und 4-Halogen-cyclohexylmethyl, besonders bevorzugt Methyl, Ethyl, Methoxy, Ethoxy, Fluor und/oder Chlor ein- bis dreifach substituiertes C₆- bis C₁₂-Cycloalkyl-alkyl, wie 2-Methoxycyclohexyl-methyl, 2-Methylcyclohexyl-methyl und 2-Chlorcyclohexyl-methyl,
- R¹, R²: - C₃- bis C₂₀-Alkoxycarbonyl-alkyl, bevorzugt C₃- bis C₁₂-Alkoxycarbonyl-alkyl, besonders bevorzugt C₃- bis C₈-Alkoxycarbonyl-alkyl wie Methoxycarbonyl-methyl, Methoxycarbonylethyl, Methoxycarbonylpropyl, Methoxycarbonylbutyl und Methoxycarbonylpentyl,
- C₅- bis C₃₀-Acetalalkenyl, bevorzugt C₃- bis C₁₂-Acetalalkenyl wie 3,3-Dimethoxy-1-propen-1-yl, 3,3-Dimethoxy-2-methyl-1-propen-1-yl und 3,3-Dimethoxy-1-methyl-1-propen-1-yl,
- gegebenenenfalls durch C₁- bis C₈-Alkyl, C₁- bis C₈-Alkoxy und/oder Halogen ein bis fünffach substituiertes C₇-bis C₂₀-Aralkenyl, bevorzugt durch C₁- bis C₄-Alkyl, C₁-bis C₄-Alkoxy und/oder Halogen ein- bis dreifach substituiertes C₇- bis C₁₂-Aralkenyl, wie 2-Phenylethenyl, 2-(4-Methyl-phenyl)ethenyl, 2-(4-Chlorphenyl)ethenyl und 3-Phenyl-2-propen-1-yl, besonders bevorzugt Methyl, Ethyl, Methoxy, Ethoxy, Fluor und/oder Chlor ein- bis dreifach substituiertes C₈- bis C₁₂-Phenylalkenyl,
- Hetaryl, z.B. Fünfring-Heteroaromaten, enthaltend ein Sauerstoff- oder Schwefelatom wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl,
- gemeinsam eine C₂- bis C₈-Alkylenkette wie -(CH₂)-, -(CH₂)₂-, -(CH₂)₃-, -(CH₂)₄-, -(CH₂)₅-, -(CH₂)₆-, -(CH₂)₇-, -(CH₂)₈-, bevorzugt -(CH₂)₃-, -(CH₂)₄-, - (CH₂)₅- und -(CH₂)₆-, besonders bevorzugt -(CH₂)₄- und -(CH₂)₅-,
- einer der Substiuenten R¹ oder R² Wasserstoff,
- R³: Wasserstoff.

Die β-Hydroxycarbonsäureester I sind geeignete Zwischenprodukte zur Herstellung von Pflanzenschutz- und Pharmawirkstoffen (Synthesis, 571 bis 590 (1989)).

### Beispiele

### Allgemeine Reaktionsgleichung

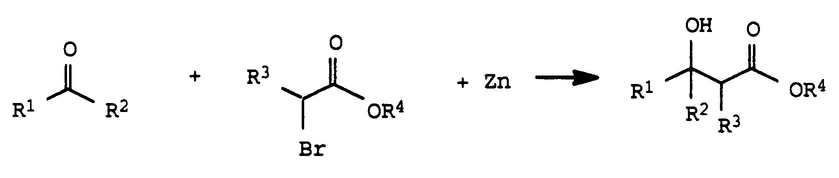

### Allgemeine Arbeitsvorschrift:

In einem 1-Liter Dreihalskolben mit Rückflußkühler, Tropftrichter, Innenthermometer und Rührer werden 42.5 g ( 0.65 mol ) Zinkpulver und 200 bis 300 mg Iod in 125 ml Methylenchlorid unter schnellem Rühren vorgelegt. Man erwärmt, bis das Methylenchlorid am Rückfluß siedet, und tropft eine Mischung aus 400 mmol α-Bromcarbonsäureester und 375 mmol Aldehyd oder Keton zu. Sobald sich die durch Iod gefärbte Lösung entfärbt, wird die Wärmequelle entfernt und die Zutropfgeschwindigkeit so reguliert, daß die Reaktionslösung gleichmäßig weitersiedet. Nach beendetem Zutropfen wird 1 Stunde unter Rückfluß erwärmt. Man versetzt mit 250 ml Methyl-tertiär-butylether und hydrolysiert bei 0 - 10°C mit 250 ml 2 N Schwefelsäure (für säureempfindliche Substrate 2 N Citronensäure). Die organische Phase wird abgetrennt, über Natriumsulfat getrocknet und eingeengt. Vakuumdestillation liefert die reinen Reformatskyprodukte wie in der Tabelle angegeben. Feste Carbonylverbindungen werden in wenig Methylenchlorid gelöst.

**Tabelle**

| Beispiel Nr. | R¹ | R² | R³ | R⁴ | Ausbeute |
|---|---|---|---|---|---|
| 1 | Et- | H | H | Et | 88 % |
| 2 | Bu- | H | H | Et | 87 % |
| 3 | Iso-Pr- | H | H | Et | 88 % |
| 4 | Ph- | H | H | Et | 95 % |
| 5 | 4-ClPh- | H | H | Et | 95 % |
| 6 | 4-MeOPh- | H | H | Et | 94 % |
| 7 | 4-MeOPh- | H | Me | Et | 83 % |
| 8 | Me-CH=CH- | H | H | Et | 91 % |
| 9 | Ph-CH=CH- | H | H | Et | 92 % |
| 10 | Furyl- | H | H | Et | 95 % |
| 11 | Et- | Me | H | Et | 89 % |
| 12 | Nonyl- | Et | H | Et | 92 % |
| 13 | -(CH₂)₅- | - | H | Me | 70 % |
| 14 | Ph- | Me | H | Et | 75 % |
| 15 | Ph- | Ph | H | Et | 80 % |
| 16 | MeOOC-(CH₂)₄- | H | H | Me | 91 % |
| 17 | (RO)₂CH-C(CH₃)=CH-* | H | H | Me | 69 % |
| 18 | (RO)₂CH-CH=C(CH₃)-* | H | H | Me | 84 % |

| | | | | | |
|---|---|---|---|---|---|
| * (RO)₂ = -O-CH₂-C(CH₃)₂-CH₂-O- : Neopentylglykolacetal | | | | | |

Analog der allgemeinen Vorschrift wurde in den Beispielen 19 bis 22 verfahren, jedoch wurde zuerst der α-Bromcarbonsäureester und anschließend das Elektrophil zugetropft.
R² = H, R³ = H, R⁴ = Me

| Beispiel Nr. | R¹ | Ausbeute |
|---|---|---|
| 19 | 2-Thienyl | 65 % |
| 20 | 3-Thienyl | 70 % |
| 21 | (CH₃)₂C=CH-CH₂-CH₂-C(CH₃)=CH- | 80 % |
| 22 | OHC-C(CH₃)=CH-CH=CH-CH=C(CH₃)- | 83 %**) |

| | | |
|---|---|---|
| **) Produkt: CH₃OCO-CH₂-CH(OH)-C(CH₃)=CH-CH=CH-CH=C(CH₃)-CH(OH)-CH₂-COOCH₃ unter Verwendung von 2 Äquivalenten BrCH₂-COOCH₃ | | |

## Patentansprüche

1. Verfahren zur Herstellung von β-Hydroxycarbonsäureestern der allgemeinen Formel I 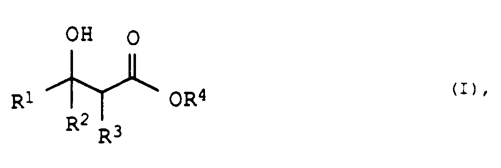 in der
R¹, R² gegebenenfalls durch C₁- bis C₈-Alkoxy und/oder Halogen substituiertes C₁- bis C₂₀-Alkyl, C₃- bis C₂₀-Alkoxycarbonyl-alkyl, C₂- bis C₂₀-Alkenyl, C₅- bis C₃₀-Acetalalkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₇- bis C₂₀-Aralkenyl, Hetaryl, C₃- bis C₂₀-Cycloalkyl, C₄- bis C₂₀-Cycloalkyl-alkyl oder gemeinsam eine C₂- bis C₈-Alkylenkette und R¹ oder R² Wasserstoff,
R³ Wasserstoff, C₁- bis C₂₀-Alkyl, C₂- bis C₂₀-Alkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₃- bis C₂₀-Cycloalkyl oder C₄- bis C₂₀-Cycloalkyl-alkyl,
R⁴ C₁- bis C₂₀-Alkyl, C₂- bis C₂₀-Alkenyl, C₇- bis C₂₀-Aralkyl, C₃- bis C₂₀-Cycloalkyl oder C₄- bis C₂₀-Cycloalkyl-alkyl,
bedeuten, durch Umsetzung von Carbonylverbindungen der allgemeinen Formel II 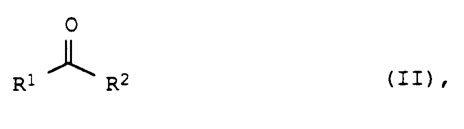 in der R¹ und R² die oben genannten Bedeutungen hat, mit α-Bromcarbonsäureestern der allgemeinen Formel III 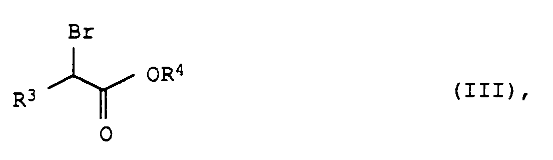 in der R³ und R⁴ die oben genannten Bedeutungen hat, dadurch gekennzeichnet, daß man die Umsetzung mit Zink in Methylenchlorid bei Temperaturen von 0 bis 50°C durchführt.

2. Verfahren zur Herstellung von α-Bromozinkcarbonsäureestern der allgemeinen Formel IV 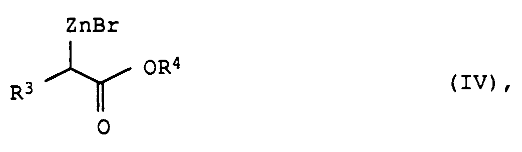 in der
R³ Wasserstoff, C₁- bis C₂₀-Alkyl, C₂- bis C₂₀-Alkenyl, gegebenenenfalls durch C₁- bis C₈-Alkyl, C₁- bis C₈-Alkoxy und/oder Halogen ein bis fünffach substituiertes Aryl, C₇- bis C₂₀-Aralkyl, C₃- bis C₂₀-Cycloalkyl oder C₄- bis C₂₀-Cycloalkyl-alkyl,
R⁴ C₁- bis C₂₀-Alkyl, C₂- bis C₂₀-Alkenyl, C₇- bis C₂₀-Aralkyl, C₃- bis C₂₀-Cycloalkyl oder C₄- bis C₂₀-Cycloalkyl-alkyl,
bedeuten, aus α-Bromcarboncarbonsäureestern der allgemeinen Formel III nach Anspruch 1, dadurch gekennzeichnet, daß man die Umsetzung mit Zink in Methylenchlorid bei Temperaturen von 0 bis 50°C durchführt.

3. Verwendung von Methylenchlorid zur stabilen Lagerung von α-Bromozinkcarbonsäureestern der allgemeinen Formel IV nach Anspruch 2.

## Claims

1. A process for preparing β-hydroxycarboxylic esters of the general formula I 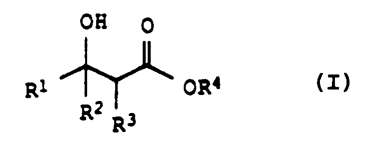 where
R¹ and R² are each unsubstituted or C₁-C₈-alkoxy- and/or halogen-substituted C₁-C₂₀-alkyl, C₃-C₂₀-alkoxy-carbonyl-alkyl, C₂-C₂₀-alkenyl, C₅-C₃₀-acetal-alkenyl, unsubstituted or C₁-C₈-alkyl-, C₁-C₈-alkoxy- and/or halogen- monosubstituted to -pentasubstituted aryl, C₇-C₂₀-aralkyl, C₇-C₂₀-aralkenyl, hetaryl, C₃-C₂₀-cycloalkyl, C₄-C₂₀-cycloalkyl-alkyl or together a C₂-C₈-alkylene chain and R¹ or R² is hydrogen,
R³ is hydrogen, C₁-C₂₀-alkyl, C₂-C₂₀-alkenyl, unsubstituted or C₁-C₈-alkyl-, C₁-C₈-alkoxy- and/or halogen- monosubstituted to -pentasubstituted aryl, C₇-C₂₀-aralkyl, C₃-C₂₀-cycloalkyl or C₄-C₂₀-cycloalkyl-alkyl,
R⁴ is C₁-C₂₀-alkyl, C₂-C₂₀-alkenyl, C₇-C₂₀-aralkyl, C₃-C₂₀-cycloalkyl or C₄-C₂₀-cycloalkyl-alkyl,
by reacting carbonyl compounds of the general formula II 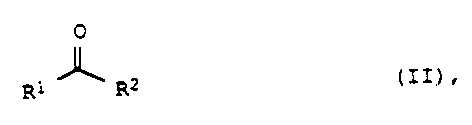 where R¹ and R² are each as defined above, with α-bromocarboxylic esters of the general formula III 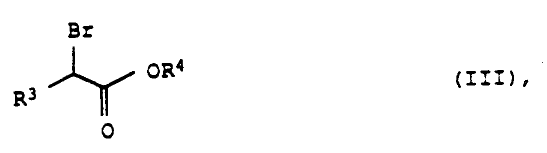 where R³ and R⁴ are each as defined above, characterized in that the reaction is carried out with zinc in methylene chloride at temperatures from 0 to 50°C.

2. A process for preparing α-bromozinccarboxylic esters of the general formula IV 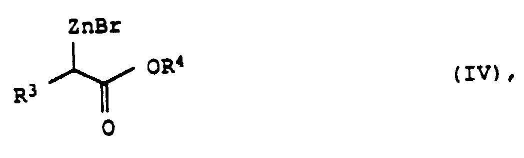 where
R³ is hydrogen, C₁-C₂₀-alkyl, C₂-C₂₀-alkenyl, unsubstituted or C₁-C₈-alkyl-, C₁-C₈-alkoxy- and/or halogen- monosubstituted to -pentasubstituted aryl, C₇-C₂₀-aralkyl, C₃-C₂₀-cycloalkyl or C₄-C₂₀-cycloalkyl-alkyl,
R⁴ is C₁-C₂₀-alkyl, C₂-C₂₀-alkenyl, C₇-C₂₀-aralkyl, C₃-C₂₀-cycloalkyl or C₄-C₂₀-cycloalkyl-alkyl,
from α-bromocarboncarboxylic esters of the general formula III according to claim 1, characterized in that the reaction is carried out with zinc in methylene chloride at temperatures from 0 to 50°C.

3. The use of methylene chloride for stable storage of α-bromozinccarboxylic esters of the general formula IV according to claim 2.

## Revendications

1. Procédé de préparation d'esters d'acides β-hydroxycarboxyliques de la formule 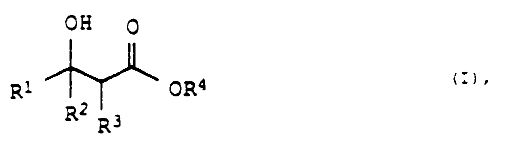 dans laquelle
R¹ et R² représentent un radical acétalalcényle en C₅ à C₃₀, alcényle en C₂ à C₂₀, alcoxycarbonylalkyle en C₃ à C₂₀, alkyle en C₁ à C₂₀, éventuellement substitué par des radicaux alcoxy en C₁ à C₈ et/ou des atomes d'halogènes, un radical cycloalkylalkyle en C₄ à C₂₀, cycloalkyle en C₃ à C₂₀, hétaryle, aralcényle en C₇ à C₂₀, aralkyle en C₇ à C₂₀, aryle, éventuellement une à cinq fois substitué par des radicaux alkyle en C₁ à C₈, alcoxy en C₁ à C₈ et/ou des atomes d'halogènes, ou forment en commun une chaîne alkylène en C₂ à C₈ et R¹ ou R² représente un atome d'hydrogène,
R³ représente un atome d'hydrogène, un radical alkyle en C₁ à C₂₀, alcényle en C₂ à C₂₀, un radical cycloalkylalkyle en C₄ à C₂₀, cycloalkyle en C₃ à C₂₀, aralkyle en C₇ à C₂₀ ou aryle, éventuellement substitué de une à cinq fois par les radicaux alkyle en C₁ à C₈, alcoxy en C₁ à C₈ et/ou des atomes d'halogènes,
R⁴ représente un radical alkyle en C₁ à C₂₀, alcényle en C₂ à C₂₀, aralkyle en C₇ à C₂₀, cycloalkyle en C₃ à C₂₀, ou cycloalkylalkyle en C₄ à C₂₀,
par la réaction de composés carbonylés de la formule générale II 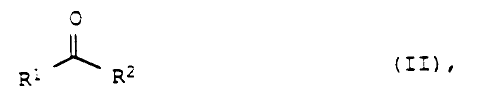 dans laquelle R¹ et R² possèdent les significations qui leur ont été précédemment attribuées, avec des esters d'acides α-bromocarboxyliques de la formule générale III 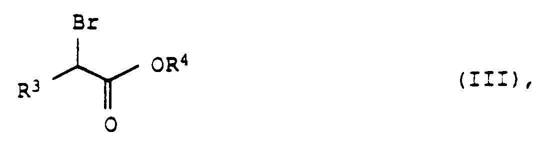 dans laquelle R³ et R⁴ possèdent les significations qui leur ont été précédemment attribuées,
caractérisé en ce que l'on entreprend la réaction avec du zinc dans du chlorure de méthylène à des températures de 0 à 50°C.

2. Procédé de préparation d'esters d'acides α-bromozincocarboxyliques de la formule générale IV 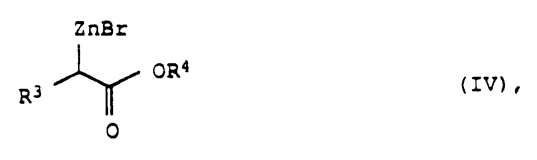 dans laquelle
R³ représente un atome d'hydrogène, un radical alkyle en C₁ à C₂₀, alcényle en C₂ à C₂₀, un radical cycloalkylalkyle en C₄ à C₂₀, cycloalkyle en C₃ à C₂₀, aralkyle en C₇ à C₂₀, ou aryle, éventuellement de une à cinq fois substitué par des radicaux alkyle en C₁ à C₈, alcoxy en C₁ à C₈ et/ou des atomes d'halogènes,
R⁴ représente un radical alkyle en C₁ à C₂₀, alcényle en C₂ à C₂₀, aralkyle en C₇ à C₂₀, cycloalkyle en C₃ à C₂₀, ou cycloalkylalkyle en C₄ à C₂₀,
à partir d'esters d'acides α-bromocarboxyliques de la formule générale III suivant la revendication 1,
caractérisé en ce que l'on entreprend la réaction avec le zinc dans du chlorure de méthylène à des températures de 0 à 50°C.

3. Utilisation du chlorure de méthylène pour la conservation ou le stockage stable d'esters d'acides α-bromozincocarboxyliques de la formule IV suivant la revendication 2.
